Europäisches Patentamt

(19) European Patent Office

Office européen des brevets

(11) Publication number: 0 018 805

A2

(12) EUROPEAN PATENT APPLICATION

(21) Application number: 80301375.4

(22) Date of filing: 28.04.80

(51) Int. Cl.³: A 61 B 19/00

(30) Priority: 01.05.79 GB 7915094

(43) Date of publication of application:
12.11.80 Bulletin 80/23

(84) Designated Contracting States:
BE DE FR IT LU NL

(71) Applicant: Douglas, Andrew Stewart McNeil
The Cottage
Guiting Power Gloucestershire(GB)

(72) Inventor: Douglas, Andrew Stewart McNeil
The Cottage
Guiting Power Gloucestershire(GB)

(74) Representative: Davies, Arthur Raymond et al,
27, Imperial Square
Cheltenham, GL50 1RQ(GB)

(54) Apparatus for controlling exhaled breath.

(57) Apparatus for controlling exhaled breath, particularly for use by surgeons, comprises a face shield 10 supported in a position to cover only a part of the face of a user, adjacent the nose and mouth, whereby the interior of the face shield will, in use, received exhaled breath from the nose and mouth of the user. A gap or aperture is provided for the ingress of ambient air into the interior of the shield, and suction conduits 13, 22 lead from outlets 12 in the shield to a small battery-operated suction pump 14, carried on a belt 15 worn by the user. In use, the suction pump 14 withdraws exhalation from the interior of the shield 10, through the outlets 12 and conduits 13, 22, and expels the exhalation into the ambient atmosphere at a desired location, for example from a downwardly facing exhaust aperture 16 on the suction pump 14.

- 1 -

"Apparatus for Controlling Exhaled Breath"

The invention relates to apparatus for controlling exhaled breath, and particularly for controlling the breath of members of surgical operating teams to protect patients undergoing surgery from pathogens exhaled by the operating team.

During normal respiratory activity, breath is exhaled through the nose in fast-moving narrow jets whereas breath exhaled through the mouth extends over a wider area and has slower movement. These phenomena can be studied by means of cine-photography within a Schlieren beam. The normal protective device worn by members of a surgical operating team is a fabric mask which is held in position by tapes so as closely to embrace the nose and mouth of the wearer. Schlieren beam studies of the flow of exhaled breath, using such a mask, show that such masks are generally ineffective and may actually assist in the spread of the exhalation. It is found that a large proportion of the exhaled breath escapes around the periphery of the mask which therefore has the effect of causing a proliferation of contaminated convection currents upon which are carried epithelial scales and dust particles containing bacteria. In addition, not all of the wearer's exhalation is dispersed through the fabric of the mask, so that the wearer is obliged to inhale less than the optimum supply of oxygen with each successive breath. The carbon dioxide contamination of the inhaled air is

- 2 -

therefore high, and this may lead, over a prolonged period, to loss of efficiency and concentration due to a low oxygen supply.

Efforts have been made to overcome these deficiencies of conventional masks, and to obtain complete protection against contamination for patients undergoing surgery, by totally enclosing the surgeon's head in a hood which is integral with the operating gown and has a transparent front panel, a suction conduit leading from the interior of the hood to draw exhaled air from the region of the wearer's face. The suction conduit, which is flexible, is connected to a fixed suction outlet, usually in the wall of the operating theatre.

Such equipment, although effective in preventing contamination of the patient, has serious disadvantages. The necessity of wearing an enveloping hood and of trailing a suction conduit coupled to a fixed outlet seriously restricts the freedom of movement of the surgeon and affects his concentration on the operation, which must be viewed through the transparent panel at the front of the hood. The complex assembly is uncomfortable to wear, and tiring when used continuously over a long period, as must often be the case.

The present invention sets out to provide an effective apparatus for controlling exhaled breath which is comfortable to wear and which does not have the deficiencies of ordinary fabric face masks or the disadvantages of the more complex equipment referred to above.

According to the invention there is provided apparatus for controlling exhaled breath, comprising a face shield, means for supporting the face shield in a position to cover only a part of the face of a user, adjacent the nose and mouth, whereby the interior of the face shield will, in use, receive exhaled breath from the nose and mouth of the user, means on the face shield defining at least one aperture for the ingress of ambient air into the interior thereof, at least one outlet from the interior of the face shield, and a suction conduit leading from said outlet to suction means for withdrawing exhalation from the interior of the face shield, through said outlet and conduit, and expelling the exhalation into the ambient atmosphere at a desired location.

Although the end of the suction conduit remote from the face shield may, if required, be connected to a fixed outlet from the operating theatre, it is normally sufficient for an exhaust aperture from the suction means to be located at the rear of the user. Means may be provided for carrying the suction means on the body of the user, so that the apparatus is entirely self-contained and the user may move freely about when wearing it. For example, the suction means may be mounted on a belt so as to be located behind the user when in use. Such an arrangement is particularly suitable for use in operating theatres where the operating area is protected against contamination by a continuous downwardly and outwardly directed flow of sterile air, since directing the exhalations from the surgeon in a direction away from the operating area is normally all that is necessary to ensure that the exhalations become entrained in the sterile airstream after the airstream leaves the operating area. It will be appreciated that, without the apparatus according to the invention, it is possible for the exhalations to become entrained in the airstream before it reaches the operating area and thus contaminate the patient.

The suction means may comprise a rotary impeller, and the rotary impeller may be driven by an electric motor which is battery powered.

Preferably the suction means is shrouded by sound absorbent material.

The aforesaid aperture for the ingress of ambient air into the face shield may be at least one one-way aperture formed through the material of the shield adjacent the user's nose and mouth, or it may be defined by a gap between a part of the face shield and the face of the user, when the apparatus is in use.

There may be provided two outlets from the interior of the face shield, and two conduits leading from the outlets respectively to the suction means. For example, the outlets may be disposed in portions of the face shield which, in use, are on opposite sides of the user's face, so that the conduits may pass over the opposite shoulders respectively of the user.

The face shield may be formed from plastics material, preferably transparent plastics material.

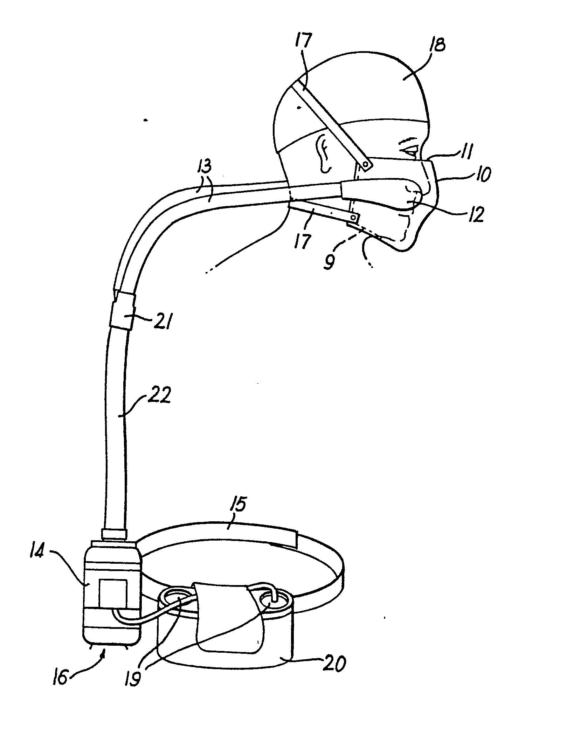

The following is a detailed description of an embodiment of the invention, reference being made to the accompanying drawing which is a diagrammatic perspective view of apparatus according to the invention.

In the embodiment shown in the drawing, the apparatus comprises a face shield 10 which is moulded from rigid transparent plastics material and is shaped to extend around the lower front portion of the user's face adjacent the nose and mouth.

The sides of the moulded face shield contact the sides of the user's cheeks and jaw, and the shield is also shaped to extend beneath the user's chin as shown. A plastics bead 9 is provided along the edge portion of the shield, where it engages the user's face and jaws so as to provide a substantially air-tight seal as well as providing a comfortable fit. Alternatively or additionally, foam rubber or plastics strips may be provided along the interior edge portions of the shield.

The face shield 10 is provided with detachable straps 17 which may pass around the back of the user's head and neck or may be connected to a headcover 18 worn by the user.

As will be seen from the drawing, the curved front face of the shield is spaced from the user's nose and mouth and there is a gap between the upper edge 11 of the shield and the user's face to provide an aperture for ingress of ambient air. Where it is preferred that the shield should fit closely to the upper face, covering the nose and mouth, suitable apertures having a non-return facility, and through which air can be drawn, may be provided through the material of the shield. Filters may be located across the inlet gap or aperture.

At each side of the face shield 10 there is provided an outlet coupling member 12 which is preferably integrally moulded from the material of the shield. Alternatively, the coupling members may be separately formed members clipped or otherwise secured to the shield. Each outlet coupling member 12 communicates with the interior of the face shield and is coupled to one end of a flexible suction conduit 13. The two conduits 13 from opposite sides of the face shield pass over the user's shoulders and are connected by a Y-coupling 21 to a conduit 22 which extends down his back to a miniature air suction pump 14 of the kind comprising a rotary impeller driven by an electric motor and contained within a housing carried by a belt 15 worn by the user. A single outlet from the air suction pump is indicated at 16 and projects downwardly and rearwardly. A filter may be provided across the outlet 16.

The air suction pump 14 may be of any known miniature type and is driven by a battery-powered electric motor so that the entire apparatus may be self-contained. The batteries 19 are then carried in a pouch 20 secured on the belt 15 adjacent the pump housing. The batteries are preferably of the rechargeable type so that they may be recharged when the apparatus is not in use. For example, in a hospital where a number of sets of apparatus are likely to be used there may be provided a multiple charging unit in which the batteries of all the sets of apparatus may be connected together and recharged overnight.

Although suitable miniature air pumps are available which are quiet when running, the casing for the pump may, if required, be shrouded by a sound absorbing material so that the slight sound of the pump does not distract the user.

In use, exhaled breath from the user passes into the interior of the face shield from where it is sucked through the outlet coupling members 12 and conduits 13 and 22 by the air suction pump 14, to be directed downwardly from the outlet 16. The action of the air suction pump 14 causes a reduction in pressure in the lower part of the face shield 10. Fresh air therefore passes downwardly into the interior of the face shield through the gap between the upper edge 11 and the user's face, or through apertures in the shield, where such are provided. In use, the inner surface of the face shield is preferably just touching, or almost touching, the user's nose leaving gaps for ingress of air on either side of the nose.

In an alternative arrangement, not shown, the face shield may be extended downwardly so that its lower portion rests on the lower part of the user's neck or upper part of his chest. In this case the lower part of the shield may be mounted on the user's clothing or on a harness which fits over his shoulders. For example the lower part of the shield may be connected to the clothing by "Velcro".

In this alternative embodiment, the face shield may be connected to the user's head or headcover 18 by straps, as in the illustrated arrangement, but the upper strap can also be omitted if required, the face shield then being supported only by the strap passing around the user's neck. In this case it will be appreciated that the user may then turn, raise and lower his head relative to the face shield which will remain stationary. This facility, besides giving an added feeling of freedom to the user, may be useful in circumstances where the face shield is required by the user when his head is lowered, for example to perform an operation, but is not otherwise required when the head is raised.

In either of the above arrangements according to the invention, further apertures may be provided through the material of the face shield to provide extra inlets for the ingress of fresh air or to permit speech to be more clearly heard through the face shield. In the case where such apertures are provided for the ingress of fresh air, these are preferably one-way apertures and in this case also the upper edge of the face shield may be shaped to fit more closely to the contours of the user's face although it will be appreciated that in this case the apparatus will be less comfortable to wear and will feel more restrictive.

Although not limited to such use, the apparatus is, as mentioned earlier, particularly suitable for use in combination with an operating theatre system in which the operating area lies in a downwardly and outwardly flowing stream of sterile air, and adds to the effectiveness of such a system.

- 8 -

## CLAIMS

1.        Apparatus for controlling exhaled breath, comprising a face shield, means for supporting the face shield in a position to cover only a part of the face of a user, adjacent the nose and mouth, whereby the interior of the face shield will, in use, receive exhaled breath from the nose and mouth of the user, means on the face shield defining at least one aperture for the ingress of ambient air into the interior thereof, at least one outlet from the interior of the face shield, a suction conduit leading from said outlet, and suction means for withdrawing exhalation from the interior of the face shield, through said outlet and conduit, and expelling the exhalation into the ambient atmosphere at a desired location.

2.        Apparatus according to claim 1, further comprising means for carrying the suction means on the body of the user.

3.        Apparatus according to claim 2, wherein the suction means has an exhaust aperture located at the rear of the user.

4.        Apparatus according to claim 3, wherein the suction means are mounted on a belt so as to be located behind the user when in use.

5.        Apparatus according to any of claims 1 to 4, wherein the suction means comprise a rotary impeller.

6.        Apparatus according to claim 5, wherein the rotary impeller is driven by an electric motor which is battery powered.

7.        Apparatus according to any of claims 1 to 6, wherein the aforesaid aperture for the ingress of ambient air into the face shield is defined by a gap between a part of the face shield and the face of the user, when the apparatus is in use.

8.        Apparatus according to any of claims 1 to 7, wherein the aforesaid aperture for the ingress of ambient air into the face shield comprises at least one one-way aperture provided in the shield adjacent the user's nose
and mouth.

9.       Apparatus according to any of claims 1 to 8,
wherein there are provided two outlets from the interior
of the face shield, and two conduits leading from the
outlets respectively to the suction means.

10.      Apparatus according to claim 9, wherein said
outlets are disposed in portions of the face shield which,
in use, are on opposite sides of the user's face, so that
the conduits may pass over the opposite shoulders respec-
tively of the user.

1/1

0018805